# EUROPEAN PATENT APPLICATION

(11) **EP 1 426 015 A1**
(43) Date of publication of application: **09.06.2004**
(21) Application number: 04100633.9
(22) Date of filing: 21.12.1998
(51) Int. Cl.: A61B 17/34, A61B 19/00

(54) **Targeting device**

(30) Priority: 31.12.1997 US 2561
(62) Divisional of application: 98959090.6
(71) Applicant: Koninklijke Philips Electronics N.V., 5621 BA Eindhoven (NL)
(72) Inventor: MARTIN, Alain, J., 5600 AE, Eindhoven (NL); VAN VAALS, Johannes, J., 5600 AE, Eindhoven (NL)
(74) Representative: Cohen, Julius Simon

(57) **Abstract**

A targeting device comprises a frame (61) including a base plate (64) and a guide plate (63) which carry the moveable guide. The targeting device has moveable guide (62) to receive an MR visible indicator (90), in particular a MR visible pen or an MR visible needle. The targeting device of the invention achieves a more accurate and more stable guiding, e.g. along an anticipated trajectory, of the MR visible invasive device.

## Description

The invention pertains to a targeting device to receive an MR visible invasive device, in particular a MR visible pen or an MR visible needle.

According the invention the targeting device comprises a frame including a base plate and a guide plate which carry the moveable guide. The targeting device of the invention achieves a more accurate and more stable guiding, e.g. along an anticipated trajectory, of the MR visible invasive device.

These and other, more detailed, aspects of the invention will now be described and illustrated with reference to the Figures, wherein
Figure 1 shows an example of a targeting device,
Figure 2 shows a movable guide and a guide plate of the targeting device,
Figure 3 shows a base plate and a suspension of the targeting device, and
Figure 4 shows an example of an MR visible pen.

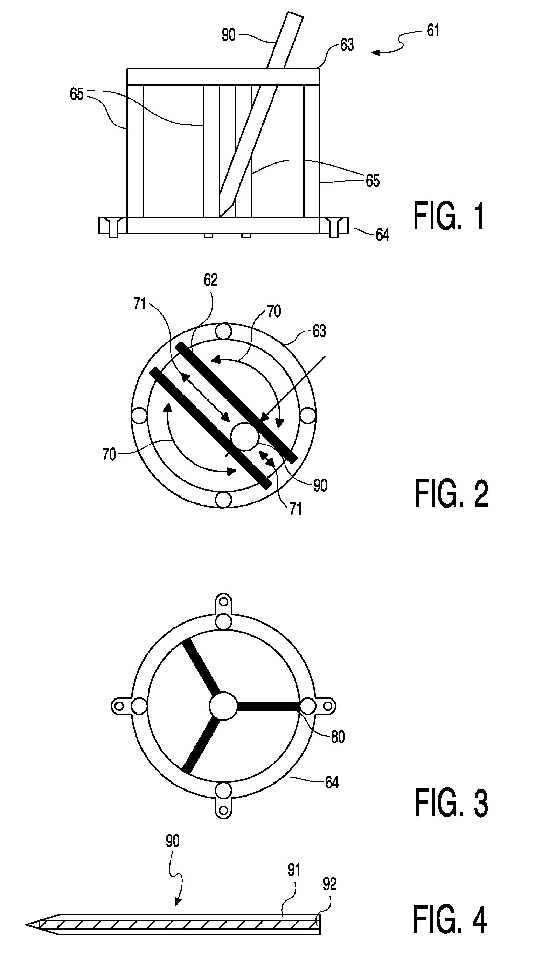

Figure 1 shows an example of the targeting device 60, comprising a frame 61 and the MR visible pen 90 for use in the method in accordance with the invention. The frame 61 comprises a moveable guide 62 for positioning the MR visible pen 90, a guide plate 63 for co-operating with the moveable guide 62 and a base plate 64. The guide plate 63 and the base plate 64 are connected to one another by parallel rods 65. The frame is made of a non-metallic material, for example carbon fibre, ceramics or a synthetic material like such as Delrin.

Figure 2 shows the moveable guide 62 and the guide plate 63. Furthermore, first arrows 70 indicate a rotation of the guide and second arrows 71 indicate the translations of the MR visible pen 90. The moveable guide 62 is capable of rotating the MR visible pen 90 with respect to a first axis perpendicular to the base plate 64 of the targeting device and a second axis parallel to the base plate 64 and of translating the MR visible pen 90 in a plane parallel to the base plate 64. The targeting device 60 can also be provided with driving means (not shown) for rotating the MR visible pen 90 with respect to the first and second axes and for translating the MR visible pen 90. The driving means may be connected to a remote control receiver for receiving commands from a remote control device controlled by the operator.

Figure 3 shows the base plate and a suspension 80 for the fixation of a distal end of the MR visible needle at the entry point. The targeting device can be fixated over the actual entry point determined by connecting it to, for example the external arm associated with the MR apparatus, or by attaching the targeting device to the skull directly. Furthermore, the targeting device 60 can be provided with a slide sleeve (not shown in the figures ) for guiding the MR visible needle.

Figure 4 shows the MR visible pen 90 comprising a housing 91 provided with a channel 92 centred along a length of the pen. The channel 92 contains an MR visible substance. The MR visible substance comprises, for example an NiCl2 solution in water, having a longitudinal relaxation time T1 of 50 ms and a transversal relaxation time T2 of 45 ms.

## Claims

1. A targeting device with a moveable guide (62) to receive an MR visible indicator (90), in particular an MR visible pen or an MR visible needle, the targeting device comprising
a frame (61) including a base plate (64) and a guide plate (63) which carry the moveable guide.

2. A targeting device as claimed in Claim 1, which allows rotation of the MR visible indicator with respect to a first axis perpendicular to the base plate and with respect to a second axis parallel to the base plate.

3. A targeting device as claimed in Claim 1 wherein the frame is made of a non-metallic material, in particular carbon fibre, ceramics or a synthetic material.

4. A targeting device as claimed in Claim 1, wherein the base plate and the guide plate are separated by parallel rods (65) that protrude transversely from the base plate (64).

5. A targeting device as claimed in Claim 2 which is provided with a driving means to actuate the rotation of the MR visible indicator.

6. A targeting device as claimed in Claim 1, wherein the base plate is provided with a suspension (80) for the fixation of the distal end of the MR visible indicator.

7. A targeting device as claimed in Claim 1 provided with a fixation to fixate the MR visible indicator over an actual entry point.

8. A targeting device as claimed in Claim 1, provided with a slide sleeve.
